# EUROPEAN PATENT APPLICATION

(11) **EP 4 748 918 A1**
(43) Date of publication of application: **27.05.2026**
(21) Application number: 24214578.7
(22) Date of filing: 21.11.2024
(51) Int. Cl.: C12M 1/00, C12M 1/36

(54) **METHOD FOR ADVANCED CONTROL OF BIOCATALYTIC PROCESSES**

(71) Applicant: Delft Advanced Biofuels B.V., 2613 AX Delft (NL)
(72) Inventor: OUDSHOORN, Arjan, 2613 AX Delft (NL); VAN LOTRINGEN, Marion, 2613 AX Delft (NL)
(74) Representative: V.O.

(57) **Abstract**

The present disclosure relates to a method for operating an integrated multiphase biocatalytic reactor system comprising a biocatalytic reaction zone and a separation zone, wherein in said method an output variable of the system, related to the produced organic substance is controlled. The control of said output variable comprises
providing a plurality of combinations of setpoints of adjustable input variables for manipulating an average residence time of the produced organic substance in the reaction zone and separation zone,
calculating a predicted value of the output variable for each of said combinations of setpoints of adjustable input parameters,
selecting a combination of setpoints of adjustable input variables for manipulating the average residence time on the basis of a comparison of the predicted values, and applying the combination of set points to the reactor system.

## Description

The present disclosure relates to a method for operating an integrated multiphase biocatalytic reactor system comprising a biocatalytic reaction zone, containing a reaction mixture, wherein an organic substance is biocatalytically produced from a substrate, the system further comprising a separation zone, wherein the reaction mixture is separated into a first fluid phase enriched in the produced substance and a second fluid phase, enriched in aqueous phase and biocatalyst, compared to the reaction mixture transferred into the separation zone.

There is a strong world-wide effort to provide more sustainable production routes, e.g. transition from fossil based substances to bio-based substances, for production of various organic substances, e.g. alcohols, esters, amino acids, carbohydrates, ethers, lipids, ketones, aldehydes, organic acids, pyridines. These substances can be used in a variety of applications for example as flavours, fragrances, cosmetic or food ingredients, nutraceutical products, bioinsecticides, specialty or commodity chemicals, or as biofuels, all as either sustainable replacement or as novel more sustainable product.

These more sustainable production routes include amongst others biocatalytic processes. It is known in the art to produce organic substances by biocatalytic processes such as fermentation and cell free conversions. In such a fermentation process, micro-organisms are used to convert a suitable substrate into an organic substance of interest. In a cell free conversion, enzymes convert a substrate into an organic substance of interest.

More specifically in the world-wide effort to produce substances more sustainably there is a need to make more lipophilic products via biocatalysis. Most lipophilic products typically have limited solubility in water due to their 'oily' nature and some are toxic/inhibiting to micro-organisms. These lipophilic products and/or by-products can be inhibiting or toxic to the micro-organism. The produced substances can be unstable under the reaction conditions in the overall reaction vessel or can stick to the micro-organism or any other surface. These effects create challenges for industrial production of these products and hamper the transition towards more sustainable industrial bio-based production. The production per process volume will be low resulting in high production cost per unit of product, creating competition disadvantage for bio-based production routes.

Lower product concentrations, i.e. relatively large stream sizes, lead to large reactor volumes and increased effort needed to perform product recovery and/or purification to end product. Additionally biocatalytic processes can generate high amount and variety of chemicals/compounds (often hundreds of chemicals) that make product recovery and further purification complex and costly. Additionally, different feed stocks may be used in the search for a cheaper carbon source. Second and third generation biomass may contain contaminants, which can be inhibiting and/or toxic to the micro-organism as well. They pose a limit on how efficient a biocatalytic process can be related to the overall production rate possible and also on product concentration being reached.

Typically, commercially applied fermentative product of organic substances, in particular lipophilic organic substances, are at present not truly continuous or limited by the nature of the way the process is set up. Either a process is currently optimised by a semi-continuous batch sequence (to service down-stream processing, which often is continuous) or is operated as a chemostat. Herein growth (mu) is set by dilution of the feed (D) and are thereby fixed to a single working point/line.

It is known in the art that biocatalytic production systems (such as fermentation production systems, experiencing diluted product streams, inhibition, equilibrium limited, and/or product stability constrained systems) benefit from in situ or integrated product removal (ISPR). Product removal can occur through a continuous phase in and out flow or only an out flow. Often this has the form of a gas stripping or liquid phase extraction, depending on product volatility or product forming its own separate liquid phase.

For the liquid phase extraction an auxiliary liquid phase may be used to extract the product from the aqueous biocatalytic phase, preferably green ,non-hazardous and non-toxic solvents. This auxiliary liquid phase is typically a compound or mixture of compounds that is at least substantially insoluble in water. The product recovery phase is usually chosen based on the organic substance that is to be recovered, i.e. it is a phase for which the produced substance has a higher affinity than for the aqueous phase in which the product recovery phase is dispersed. Thus, the organic substance of interest is removed from the aqueous phase by phase affinity difference. Suitable phases can be selected for an organic substance of interest based on common general knowledge or empirically. A further property to consider when choosing the product recovery phase is the density of the product recovery phase. A difference in density with the aqueous phase is desirable because this allows separation by gravitation or centrifuging. Particularly suitable liquids for the auxiliary liquid phase are organic liquids, in particular hydrophobic organic liquids, which form a phase separate from the aqueous phase of the reaction mixture.

A further possibility is to change from batch operation, which is still common in the fermentative production of organic substances, to continuous operation.

Continuous and/or semi continuous operation allows for control of residence time of certain fluid phases and/or substances in the system. In combination with continuous in situ substance sustainability and economic viability can be improved. WO2021/010822 and WO2024/049295 disclose devices and methods allowing the continuous fermentative production and extraction of produced product in a single apparatus.

Herein liquid-liquid phase separation by gravitational forces is applied as ISPR (ISPR, integrated in the fermentation reactor system itself, " the continuous or intermitted addition and removal of product recovery phase provides an extra degree of operational freedom, which can be used to control the effective dispersion characteristics of the product recovery phase (droplet size in case of a liquid, residence time, mass fraction of product recovery phase)".

Although these devices and methods thus allow for an additional degree of freedom in operation and allows matching of ISPR and separation capacity or requirement, the inventors realised that there is a need for advanced control of biocatalytic processes, more specifically for the biocatalytic production of lipophilic products.

In particular, the inventors realised that lipophilic products, due to their nature, introduce certain limitations in the bioprocess, which therefore lacks industrial viability and/or sustainability and can't be overcome directly with ISPR only. Using an auxiliary liquid phase for liquid-liquid extraction for controlled substance (e.g. product) removal from the aqueous stream originating from biocatalytic reaction processes introduces an additional degree of freedom. This operational freedom created by ISPR in principal opens up a new operating envelope, by lifting existing limitations, for example enabling new ways to improve a bioreactor system output variable related to the produced substance, such as the volume-specific productivity. However, improvement of such output variables of biocatalytic processes remains a key challenge in bioprocess development in particular for lipophilic products and is not solely dependent on fermentation or mass transfer and just the variable at hand, but also interconnected variables crucial for the overall performance of the production system, like e.g. yield and product concentration. Further optimisation and control of these processes is not straightforward.

The method of the present disclosure offers an advancement in the operation of a continuous production in a(n aqueous) reaction medium and separation from that reaction medium of a biocatalytically produced organic substance, in particular an advancement in the operation of continuous extractive fermentation and controlling the fermentation on multi-variable level in both input and output. The inventors found that pro-active control of conditions, which in standard fermentation are seen as an end result, e.g. product concentration, is now *a priori* input to the system. The method of the present disclosure allows to determine the desired specific operation specifically allowing usage at previously unobtainable operating conditions and/or modes of operation, feasible by the extra degree of freedom created by ISPR. The present disclosure is opening new ways for fermentation run design and optimisation, by determining new operating window(s) with a scala of possibilities, allowing a different level of optimisation of one or more key performance indicators needed for fermentation technology to be more competitive and sustainable; these consist of combining at an output level variables relevant to the produced substance, both at biocatalyst level and also at reactor, process and process integration level.

Accordingly, the present disclosure relates to a method for operating an integrated multiphase biocatalytic reactor system comprising a biocatalytic reaction zone, containing a reaction mixture, wherein an organic substance (the product) is biocatalytically produced from a substrate, the system further comprising a separation zone, wherein in said method an output variable of the system, related to the produced organic substance, is controlled. The method of the present disclosure comprises
- providing the reaction mixture, wherein the organic substance is produced using a biocatalyst, which reaction mixture comprises an aqueous phase, in which the biocatalyst is preferably dispersed or dissolved, which aqueous phase comprises the substrate for the biocatalyst, and wherein further droplets of a liquid product recovery phase, comprising the produced substance, are dispersed in the continuous aqueous phase;
- at least during an essentially steady state stage: continuously our semi-continuously feeding substrate for the biocatalyst into the reaction zone;
- at least during an essentially steady state stage: continuously our semi-continuously transferring reaction mixture from the reaction zone to the separation zone;
- separating the reaction mixture in the separation zone into an upper fluid phase, enriched in the product recovery phase comprising the produced substance and a lower fluid phase, enriched in the aqueous phase and the biocatalyst, compared to the reaction mixture transferred into the separation zone;
- at least during an essentially steady state stage: continuously our semi-continuously selectively withdrawing the enriched product recovery phase from the system or the separation zone of the system.

The control of said output variable in said method of the present disclosure comprises
(a) providing a plurality of combinations of setpoints of adjustable input variables for manipulating an average residence time of the produced organic substance in the reaction zone and separation zone. As used herein tau (τ) or average residence time refers in particular to the average hydraulic residence time of the produced organic substance from product formation in the reaction zone till removal from the separation zone. Thus, a combination defines a set point for the manipulated (adjustable) variable ` residence time'.
   The control of said output variable in said method of the present disclosure further comprises
(b) using a process operating performance model to calculate a predicted value of the output variable for each of said combinations of setpoints of adjustable input parameters;
(c) comparing the predicted values of the output variable;
(d) optionally repeating steps a), b) and c) (typically using results obtained in a previous step c for the next step a);
(e) selecting a combination of setpoints of adjustable input variables for manipulating the average residence time on the basis of said comparing in step c). The selecting may be on the basis of results of one or more steps c), typically at least including the last time step c) is performed, if step d) is performed. Setpoint(s) are typically selected based on a satisfactory process operating performance used in the comparison in step c). A combination of setpoints aims to achieve a target output variable, which can be a target output variable window/range.

The control of said output variable in said method of the present disclosure further comprises
(1) applying the combination of set points of adjustable input variables selected under step e) to the reactor system. An aim of applying such combination is achieving a target process operating performance or at least to reduce the deviation from the target process operating performance).

If desired, the control of said output variable in said method of the present disclosure comprises monitoring the output variable. If desired, said control further comprises adjusting one or more of the adjustable input variables setpoint(s) if the value of the monitored output variable deviates from a target value of the output variable or is outside a target value range of the output variable, or repeating one or more of steps a) - f).

### BRIEF DESCRIPTION OF FIGURES:

Figure 1 illustrates the relationship between input and output variables around a (bio)chemical process (source: *Stephanoporclos* Fig. 2.1).
Figure 2 is illustrative to an embodiment wherein ISPR impacts the average product residence time and its distribution.
Figure 3 shows monitoring results for a model example of the disclosure wherein 2-phenylethanol (2PE) is produced.

The in situ substance removal uncouples the operation of the aqueous phase wherein the biocatalytic reaction take place from the organic product recovery phase. The levels of substances, e.g. produced organic product and recovery phase, can thereby be controlled independently. This allows more 'steady state' reaction conditions, more 'steady state concentration profiles of produced substance, or both. Uncoupling of aqueous and organic liquid fractions allows uncoupling of produced substance, from growth conditions and maintenance conditions. The specific substance production rate can now be freely chosen and not constrained by the batch overall mass balance. So for example, the dilution rate in a chemostat, having to be equal to the growth rate is no longer a requirement as the aqueous dilution rate can now be chosen and is not determined by the volume mass balance. Additionally, operation at low growth and high substance production rates, under low maintenance requirements, is now also possible. These freedoms result in improved process performance, for instance optimisation of yield.

The method of the present disclosure makes use of controlling an output variable; the method can benefit in particular from an improvement in one or more of the following.
(1) Substance inhibition/ toxicity control, for example inhibition of biocatalyst by the produced substance, e.g. in case of terpenes, alcohols, aldehydes; or inhibition of biocatalyst due to a contaminant in a feedstock.
(2) Oily products recovery and/or emulsification control: continuous removal of oily products prevents emulsification, prolongs production and increases available production volume. For example in case of the production of long-chain fatty acids. In particular in such embodiment, the produced substance may form its own recovery phase, without the need of a separate extractant.
(3) Substance degradation control. Biocatalytically produced substances that are instable in the aqueous phase can be stabilised in the organic product recovery phase, and residence time of such substance in the reactor is minimised. This is for instance useful in the production of vitamins, aldehydes, dyes.
(4) Excretion or lifting equilibrium limitation. Continuous extraction increases concentration gradient and therefore enhanced flux across cell membranes. Product transfer to the organic phase maximises extent of reaction. This is in particular interesting for lipophilic product that are not readily aqueously soluble, like most bio-oils, fatty acids and fatty alcohols, and for enzyme-catalysed conversions in Liquid-Liquid systems.
(5) Flash point lowering due to shift in equilibria system. By addition of the organic extractive phase the liquid-liquid-gas equilibria of products can be set to specific conditions throughout the fermentation. This allows, e.g., to operate at non-explosive conditions, to have lower vapor content of product, to reduce/mitigate stripping losses or the like. This is especially beneficial in case of the production of substances with a flashpoint below 50 °C.

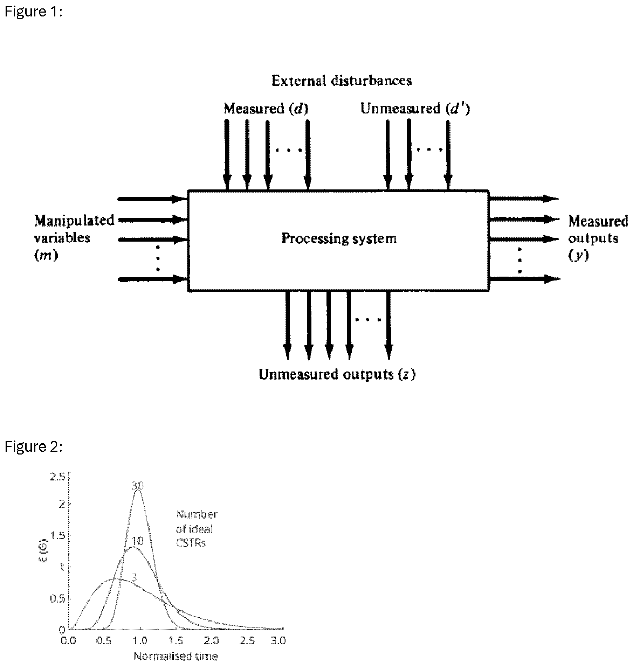

Specific embodiments in which the method of the present disclosure is advantageously applied, include:
(1) Product concentration control (mass balances). This can be applied in order to run at optimal overall volumetric productivity/growth rate and yield combinations. Key elements are to have A) the microbial conversion modelled as function of growth, maintenance and product inhibition and B) resolution and control of the (product) mass balance and hold-up of the relevant phases. This combination exceeds the current best practices applied in the art.
(2) With reference to Figure 2, the control of the product hold-up (normalised time) in the reactor and the effective product fraction and distribution of the product residence time is brought from a complex and broad time distribution curve to a high Peclet number (E(θ)); analogous to axial dispersion the residence time of product in the reactor can be controlled. Continuous product removal (ISPR) impacts the average product residence time and its distribution and thereby allows for control of this product residence time, the residence time distribution and the impact on the process output.
   Combination of 1 and 2 (product concentration control and product hold up control) allows for enhanced cell performance by lowering the cell exposure to product as active and passive diffusion is well known to be dependent on product gradient over a membrane.
(3)Lower allowing an effective increase in product flux over the biocatalyst, as the driving force for biocatalyst diffusion is increased.
(4)Product formation, by-product formation and substrate consumption in a dynamic system is often depending on the product concentration as operational variable in time. In biotech, the product concentration dependency in cell free conversions (enzymatic reaction) is often well known. Combining the enzymatic reaction together with mass balance control enables enhanced optimisation of extend and/or rate of reaction to product and by-products. The inventors realised that the optimisation should start with determining the optimal steady state substance concentration in the multiphase reaction system based on the enzyme equilibrium constants, followed by applying a control strategy to maintain the desired equilibrium and rate. It is crucial that the mass fractions and residence time of organic and aqueous liquid phases are aligned with the chemical/enzymatic reaction, biocatalyst (enzyme or microbe) content and location.
(5)In case of a reactive gas-liquid-liquid systems, a certain work point, i.e. the concentration of specific chemicals, can be controlled by adjusting the mass balance control of the organic phase to the reaction rates. In a preferred embodiment explosive and our hazardous gas concentration are not present during operation as the mass balance is aligned with the reaction.

On bioreactor level, a method according to the present invention can be optimised from both a hardware utilisation and on operational utility consideration. For environmentally sustainable processes it is crucial to optimise energy-consumption, materials consumption; Such an optimisation can be further achieved by this disclosure by realising improved reactor and process operation is possible by for example recycling of stream(s), addition of stream(s), operating units of various sizes at various operational modes in parallel and/or series configuration, by integrating upstream processing (USP) and downstream processing (DSP) with respect to energy (heat) balance, material balance or both similar to the operational envelop and process control and optimisation for the individual biocatalytic reaction unit as previously described. In particular the present method herewith offers a useful improvement with a focus on optimising product yield and recovery, while minimising resource usage and waste generation.

The inventors further found especially that the method of the present invention offers an advantage to controlling an output variable in a process benefiting from influencing the metabolic fluxes in the cell, as metabolic pathway optimisation is achieved by creating and controlling the specific conditions in the fermentation processes. Setting the operating envelop conditions, either all the time or at a certain fraction of the time, at a certain frequency lead to a substance production yield shift away growth and/or maintenance of biocatalyst (living cells) in a fermentative organic substance production. It is further interesting that does also mean that the specific oxygen uptake requirement on substance, e.g. product, produced can now also be further optimised, as they are not independent variables. E.g. setting (by the enhanced control) of conditions in the process, determining the specific growth-rate and catalytic conversions rates desired from the perspective of the cell, often in relation to yield and product concentration), the required mass transfer of all components involved to maintain the conditions, jointly creating an enhanced production system, with optimised performance on desired multiple key performance indicators.

Generally, apart from the controlling of the output variable(s), in accordance with the present disclosure the biocatalytic production, the separation of the product recovery phase containing the produced substance and optional further down-stream process can be based on known processes, e.g. as described in prior art cited in the present disclosure in combination with the present disclosure and common general knowledge. The controlling can be based on common generally known principles regarding procures control for the chemical industry, e.g.. *Chemical Process Control, An Introduction to Theory and Practice, George Stephanopoulos, PTR Prentice Hall, 1984),* (elsewhere herein: ' *Stephanoporclos*') supplemented with the information disclosed herein.

The inventors realised that the residence time (typically the average hydraulic residence time) of a biocatalytically produced organic substance in multiphase biocatalytic reactor systems wherein both reaction and recovery are performed, can advantageously be used in controlling an output variable of the system, related to the produced organic substance, the method of the present disclosure allows the residence time to become utilisable as a advantageously controllable variable.

As is generally known in the art (see e.g. *Stephanoporclos,* Chapters 1 and 2) variables in a chemical process include input variables, which denote the effect of the surroundings on the chemical process and output variables, which denote the effect of the process on the surroundings, See also Figure 1, based on Fig. 2.1 of *'Stephanopoulos'.* The input variables can be classified as manipulated variables (a.k.a. adjustable variables) if their values can be adjusted freely by a human operator or a control mechanisms. Input variables of which the values are not the result of a direct adjustment of the variables' value are referred to in the art as disturbances. Output variables can be distinguished in measured output variables, if their values are known by directly measuring them and unmeasured output variables, if they are not or cannot be measured directly.

Residence time, such as residence time of a produced substance or product recovery phase in a bioreactor system or part thereof (such as the reaction zone or separation zone), as such is not an input variable nor an output variable. It is a variable within the processing system affected by other variables. Changes in residence time can be accomplished by or by the result of changes in the input variables. Residence time can thus be manipulated by adjusting one or more adjustable variables. Thus, adjusting certain adjustable parameters may be employed to control residence time. A variable, e.g. a residence time, that is affected by an adjustment of an adjustable parameter is also referred to in the art as a controlled variable.

Compared to chemical processes, biocatalytic processes, in particular when making use of a living organism as a biocatalyst, are generally much more prone to adverse effects by changes in certain input variables and often effects are not reversible. The route to the desired setpoints is often just as important as the setpoint itself in biocatalytic processes versus chemical processes.

The present disclosure allows a further improvement in a produced substance related output variable by setting and controlling operating process parameters of the integrated multiphase biocatalytic reactor system, configuration of the integrated multiphase biocatalytic reactor system, or both.

Applying this advanced control to fermentation processes can be of particular benefit for biocatalytic processes in which control is desired of certain conditions to obtain a more optimal result by for example influencing the metabolic flux of the microbe or at higher level using resources more optimally, thereby improving yield and reducing waste streams and allowing combination of production rate and yield previously unobtainable in fermentation systems in which growth of microorganisms is allowed, but controlled by conditions. In biotechnology substrate is converted in biomass, products, energy, and undesired by-products. Control of process performance throughout all stages of the process is complex, as variables and outputs are interdependent and therefore optimisation is not just a matter of changing one variable, but rather a complex solution of a set of variables and interactions. E.g. the product concentration level of a produced inhibiting organic substance influences not only biomass growth rate but also microbial stress leading to by-products formation and reduced efficiency as the cell requires more maintenance, the chemical interactions and equilibrium concentrations between the aqueous and organic phase (i.e. the mass transfer rates due to concentration effects), and further in the process impacts downstream processing. Unlike batch fermentation processes which have limited options to control product concentration levels, control of product levels throughout the fermentation process is possible for more continuous fermentations. The method of the present disclosure not only allows optimisation from overall process performance for typical parameters in fermentation processes like yield (conversion of substrate to organic substance) and conversion rate, but also one or more of: (by)-product concentration(s), growth rate, specific biocatalyst activity, organic phase residence times, organic substance recovery rate, product recovery efficiency, energy efficiency/specific energy utilisation, equipment utilisation, raw material utilisation, waste management, turn-around time (minimisation of non-productive time), process safety, process reliability, process stability.

A specific benefit of a method according to the present disclosure is that complex process performance can be predicted by analysing a pool of input and output variables on overall process level and incorporating them into one or more models. Using one or more of these models, e.g. microbiological performance model in combination with the overall reactor mass balance model, to identify a combination of setpoints of input variables to steer the process towards a desired performance from a microbial perspective and the desired performance from an overall mass balance perspective. For example the combination of setpoints of input variables lead to specific residence times and mass transfer rates, required to maintain the desired conditions and performance.

In summary this means, for example, that both a biocatalytic kinetic model is assessed jointly with a detailed gas, liquid, liquid, solid phase mass and fluid dynamic kinetic model, which includes at minimum the dynamic exchange rates of molecular components between gas phase and liquid phase. A combination (or integration) of models allows for assessment of desired conditions. However, one must have the realisation that the determining boundary condition of these sets of equations, is the kinetic biocatalytic production and growth model of the desired strain performance under pseudo steady state conditions; this realisation is an important and non-obvious contribution of the present disclosure to the art. With this boundary condition and the realisation that overall reactor and process mass balances and biocatalytic activity can be controlled pro-actively, advanced control of the production process is now possible.

The required mass transfer now can be determined by reactor mass transfer modelling and thus follows; This rational now allows a person skilled in the art to determine the required input variables necessary to obtain the desired conditions.

This control is needed to allow fermentative and catalytic processes to be more efficient on raw materials and utilities and overall process efficiencies, predominantly production rate, in order to have sustainable production of chemicals to be enabled. Industrial production of chemicals needs to be move away from fossil resources.

In an embodiment, the method of the present disclosure comprises controlling a process targeting a certain value range for an output variable selected from the group comprising of produced organic substance recovery rate (product recovery rate), yield (conversion of substrate to the organic substance), product concentration in the organic phase, microbial (biocatalyst) activity, product recovery, liquid-liquid phase separation/flow velocity in separation zone, oxygen mass transfer rate, microbial productivity, energy efficiency, metabolic flux, raw material utilisation, waste management, turn-around time (minimisation of non-productive time), process safety, process reliability, process stability, microbial biocatalyst substrate conversion rate, microbial biocatalyst growth rate, liquid-liquid phase separation/flow velocity in separation zone, oxygen mass transfer rate, carbon dioxide production rate, oxygen conversion rate, product concentration in the aqueous phase.

In a preferred embodiment, at least the following variables are controlled: microbial substrate conversion rate, biomass growth rate, (by)-product concentration in the aqueous phase fraction in the reaction zone and organic phase residence time. This combination is in particular advantageous in order to obtain an optimisation of product yield on substrate for inhibiting products.

In a further preferred embodiment, at least the following output variables are controlled: product concentration in the organic phase and microbial activity. This has been found in particular advantageous in order to optimise energy efficiency of the overall process (including downstream processing), substrate yield to product.

In a further preferred embodiment at least the following output variables are influenced to a desired value: liquid-liquid phase separation, aqueous phase residence time by means of flow velocity through the separation zone, biocatalyst activity. Biocatalyst activity loss in the separation zone or recycle can be due to for example, shear stress, lack of nutrients/substrate (Carbon source & O₂), pH, Temperature.

Advantageously, for aerobic fermentation, the oxygen concentration in the reaction zone is controlled. Such embodiment is in particular of influence on the chosen geometry of the vessel and it's different zones in combination with total reactor volume. Preferably optimisation is a combination of process control of the output variables together with an optimised reactor geometry and volume.;

In a further preferred embodiment, at least the following output variables are controlled: biomass fractions in the separation zone, and enrichment of at least one liquid phase in the separation zone. This allows for the optimisation of aqueous phase and biocatalyst losses.

In a further preferred embodiment the controlled output variables at least include the following combinations ratio carbon dioxide production rate and oxygen conversion rate, in the form of the respiratory quotient (RQ), and aqueous phase product concentration. The output data of these variables can be made available real time and is a good indicator of the real time performance of the system, i.e. the net outcome of the complex set of current operating conditions, for example aqueous product concentration, cell activity, cell amount, substrate levels, and the dissolved oxygen concentration. Real time control of the current product yield on substrate and current reactor productivity is now possible by manipulating the product residence time (effective hold-up at specific concentrations) in the reactor in combination with monitoring the RQ and the aqueous phase product concentration. In a specific embodiment of the present disclosure, 2-phenyl ethanol is biocatalytically produced. Preferably for optimisation of the yield and productivity for 2-phenylethanol producing yeast, the RQ is in the range of 1.0 - 1.3, preferably close to 1.1, and oxygen concentration is at limited/restricted conditions (dissolved oxygen below 20% dissolved oxygen, but preferably very close to 0%), while the aqueous 2-phenylethanol concentration is in the range of 0.3 - 1.5 g/L, preferably close to 0.5 - 1.0 g/L. As co-inhibition of by-products takes place ethanol and acetate concentrations should be controlled and kept within bounds.

In particular, in a method of the present disclosure for the production of an organic substance that is inhibiting to the biocatalyst, it is useful to control the following combination of output variables : product formation rate, yield and growth rate, together with aqueous phase product concentration, organic phase residence time in reactor zone, organic phase stream removal rate from the system and overall process including energy utilisation and product recovery and purity. This has the benefit of optimizing the process as a whole, i.e. optimisation of both upstream processing (biocatalytic conversion) and downstream processing (product recovery and purification) at the same time.

The control of the output variable comprises providing a plurality of combinations of setpoints of adjustable input variables for manipulating an average residence time of the produced organic substance in the reaction zone and separation zone.

Herein a "setpoint" is the target value of the adjustable input variables. Adjustable process variables for which a setpoint can be given in step a) include, a substrate feed rate, a nutrient or co-feed feed-rate, a water make-up stream, an internal recirculation flowrate, an aeration rate, a rate of discarding reaction mixture rate from the reactor system, a transferring rate of reaction mixture into the separation zone, a withdrawal rate of the enriched product recovery phase from the separation zone, a gas feed rate of the reaction zone (such as a feed rate of air or oxygen), a characteristic mixing time, a mass transfer resistance between gas to liquid, a mass transfer and/or mass transfer resistance between liquid-liquid, an addition rate of acid/base, a substrate selection (e.g. 1st/2nd generation feed, sugars, glucose, glycerol), a concentration of composition of surface active chemicals, a water evaporation rate, a liquid level, a gas hold-up, a flow velocity, a height/length of characteristic flow zones, a pressure losses by hydraulic friction, a dispersed phase liquid droplet sizes and distribution, a substrate concentration, a selection of an organic liquid for the product recovery phase, a biocatalyst activity, a biocatalyst concentration, a biocatalyst selectivity, a temperature, a pressure, a pH, a conductivity of aqueous broth, an organic liquid phase mass fraction, an organic phase liquid residence time, an aqueous phase residence time, a microbial growth rate, a microbial washout rate; ratio of carbon dioxide production rate and oxygen conversion rate, a gas bubble size and gas bubble size distribution, a dissolved substrate or product concentration, a dissolved oxygen concentration, a dissolved carbon dioxide concentration. These process variables are predominantly directly or indirectly manipulated variables by actuation of pumps and/or valves and/or mechanical power input. Preferably, combinations are provided comprising at least two setpoints of adjustable process variables selected from the group of mass balances related process variables and biocatalytic related process variables. Combination of a set of setpoints of adjustable process variables allows at minimum optimisation and control of one defined target system performance. Preferred combinations are:
(1) a combination of microbial substrate conversion rate and growth rate, with product concentration and organic phase residence time (product on substrate yield benefits);
(2) a combination of product concentration in the organic phase and biocatalyst activity (energy efficiency of the overall process and yield of substrate to product);
(3) a combination of liquid-liquid phase separation/residence time of the aqueous phase by flow velocity through the separation zone, oxygen mass transfer rate and biocatalyst productivity (optimisation in combination with reactor geometry, including reaction zone and separation zone); liquid phase separation, surface active chemical concentrations and biomass concentration (optimisation of aqueous phase losses and biocatalyst losses);
(4) a combination of RQ and aqueous phase product concentration (impacting overall yield and reactor productivity).

In a particularly preferred method of the present disclosure a surface active chemical property, a salt property or a combination thereof is used as adjustable input variables for which a target if set. The property can be quantitative ( concentration), qualitative (choice of specific salt(s), choice of specific surface active chemical) or both. Changing the concentration or type of surface active chemical, salt or both can be used to influence separation of the aqueous phase and the (organic) product recovery phase from each other in the separation zone (or during further downstream processing). Changing the concentration or type of surface active chemical, salt or both can be used to influence extraction performance of the biocatalytically produced organic substance from the aqueous phase to the (organic) product recovery phase (in the reaction zone). Without being bound by theory, the inventors consider that a physicochemical property of an interlayer between aqueous phase and (organic) product recovery phase can be influenced by the salt or surface active chemical, thereby influencing the separation or extraction. In particular, the polarity of at the interface is considered to play a role in this.

Liquid-liquid phase separation rate in the separation zone preferably is at least 100 L/m²/h, more preferably at least 200 L/m²/h, even more preferably at least 450 L/m²/h. Flow conditions in and/or through and/or towards the separation zone are usually essentially laminar (Reynolds (Re) <2300).

Internal recirculation between the separation zone and the reaction zone is preferably close to laminar or laminar (Re<2300);
Reactor substrate to product conversion rate is preferably >1 g/L/h, more preferably 3 g/L/h, even more preferably 5-10 g/L/h. For glucose as substrate for aerobic conversions the targetted RQ is >1, preferably >3 for very reduced products (oxidation state).Gas hold-up is usually 5%-75%.

Actively flowing aqueous phase residence time in the internal recirculation from separation zone to reaction zone is usually < 2 minutes for aerobic fermentations. Usually said residence tome in the internal circulation is more than 15 sec, in particular about 30 to about 90 seconds.

Ratio organic phase product concentration to aqueous phase product concentration (weight to weight) for extractive processes is usually at least 4, preferably at least10, in particular at least 50, more in particular at least 100. Usually, said ratio is up to about 300, in particular up to 150 or up to 100. However, for products with a very low aqueous solubility and high affinity said ratio can exceed 300, and can e.g. be up to 500 or up to 1000, or even higher.

Organic phase volume fraction in the reaction zone is usually 10% or less, preferably 5 % or less. Organic phase volume fraction in the reaction zone is usually about 1 % or more, preferably about 2 % or more.

The organic phase removal from the system for industrial units per day is, on volume basis, usually at least 10% of the total system volume, preferably at 30 % of the volume, more preferably close to the total volume, such as up to 90 % or higher. For smaller scale pilot and demonstration units even larger % volumes per day, in particular up to 350%, are possible.

Preferably, in step b) a combination comprising at least two setpoints of adjustable process variables selected from the group of mass balances related process variables and biocatalytic related process variables are used. The skilled person will be able to choose a logical combination based on the present disclosure and common general knowledge, i.e. a combination of adjusted variables that are relevant for a particular output variable that is to be controlled. A combination of a set allows at minimum optimisation and control of one defined target system performance output.

The combinations of setpoints are used in step b) to calculate a predicted value of an output variable(s) that is (to be) controlled for said combinations of setpoints. For this a process operating performance model, like a flowsheeting model, is used. Suitable process operating flowsheeting models can be based on those known in the art for modelling (bio)chemical industrial processes using kinetic microbiological model and reactor mass transfer kinetic models as an input/basis for example.

In a preferred embodiment, step b) includes the use of a process operating performance model that takes inhibition of the biocatalyst, in particular a living organism, by the produced organic substance into account. Such model advantageously comprises one or more elements based on the modelling described by Straathof 'Modelling of end-product inhibition in fermentation' in Biochemical Engineering Journal 191 (2023) 108796. The author clearly realises inhibition modelling and understanding is needed to predict fermentation performance "Still, product inhibition models remain a weak part of fermentation models..." ). The author adds to the current inhibition models separate constants for substrate biomass formation rate and for substrate consumption rate for maintenance (as a function of inhibition), agreeing that optimisation is complex multivariable problem.

However, the application of the fermentation inhibition model by Straathof is limited to conventional (fed)-batch fermentation and chemostat fermentation systems. It is not including systems in which the fermentation mass balance can be controlled to further extent, such that the aqueous phase mass balance and the organic phase mass balance become more independent; In this situation the growth rate requirement for chemostat fermentation (growth rate = dilution rate on the system) is no longer a boundary condition to the fermentation process, nor does the infinite retention time and volume limits of a batch system exist. Advanced control in which the biocatalytic performance in combination with the overall mass balances over the reactor (and process) are controlled is thus required to further process optimisation. The aqueous product concentration is an important input and variable to the biocatalytic conversion model and the mass transfer models, as product formation rate, biomass growth rate and product removal rate are controlled in order to bring the system to the desired operating window for further yield optimisation. Without independent mass balance control of the aqueous and organic liquid phase the chosen setpoints are often not sustainable or possible in either batch, fed-batch of chemostat systems. The preferable situation being the product concentration remaining constant, under low biomass losses.

The predicted values of the output variables obtained by the comparison in step b) are compared in step c).

Steps a), b) and c) can be an iterative process (cf. optional step d)). On the basis of the comparison in step c) a further plurality of combinations of the setpoints of the adjustable process variables can be provided (step a)), used in the operating performance model (step b)) and compared in a further step c) with each other and optionally with predicted values of output variables in a previous iteration.

In step e), a combination of setpoints (changes) of adjustable variables, to control the output variable(s) as desired as follows from the comparison (c), is obtained by matching biocatalytic response (performance) of the system with model to predict changes to the overall mass transfer and resulting mass balances, preferably by manipulating the average residence time of phases and chemical species, by calculating the required change in process variables (flowrates, volume, etc) and the required change to the setpoints of the current actuators (pumps, valves).

Typically an overall process model, like a flowsheeting model, (incorporating microbial conversion input and mass balance kinetic model input) is used to run different cases / case studies to generate predicted outcomes per case and use the outcomes to determine the optimum operation. For example optimum operation can be an economic optimum within certain boundary conditions, e.g. product specification, environmental limits, etc.

The selected combination of setpoints of adjustable input variables is applied to the method of the present disclosure. Said selected combination used to operate the integrated multiphase biocatalytic reactor system, whereby the output variable of the system, related to the produced organic substance is controlled more effectively. In particular, applying the selected combination of setpoints to the method allows achieving a target output variable or at least to reduce a deviation from a target variable, compared to a similar method lacking the control of the output variable in accordance with the present disclosure as control of both catalytic conversion process and the overall mass balances (per species and/or phase and their related mass transfer rates) are pre-determined by modelling, applied to the system, monitored in off-set from desired output and regulated accordingly.

If desired, the controlled output variable is monitored during at least a part of the method according to the present disclosure, in particular at least during the essentially steady state stage or a part thereof. An output of the monitoring (such as measured values of the output variable) can be used to directly adapt the setpoints of the operating bioreactor system, whilst the method is continued, if needed. For this, an output of the monitoring is compared with a target value point or a target value window. If the output of the monitored output deviates more than desired from the target value point or deviates from the target value window, one or more of the adjustable input variables (such as one or more selected from those used in step a)) can be adjusted, e.g. based on the existing results of the steps a), b) and c) of the method of the present disclosure, or one or more of steps a)-f) can be repeated, in particular at least steps e) and f), more in particular at least steps a), b), c), e) and f). The method can be either performed offline with intermittent adjustments, or partial online control of certain variables or can be fully incorporated into an automatic (optionally self-learning) control system.

In an advantageous embodiment of a method according to the present disclosure conditions are controlled to steer towards improved volumetric productivity by controlling/bringing the system to optimum conditions, for the combination of the biocatalytic specific substance formation rate and the amount of biocatalyst present, like nutrient feed rate/substrate availability (e.g. O₂), substrate concentration and substance concentration.

In an advantageous embodiment of a method according to the present disclosure the liquid-liquid phase separation is optimised by controlling the reactor concentrations of specific combination of variables. Preferably these variables include one or more, in particular two or more of the following: surface active compound concentration, salt concentration, internal flow conditions and power input. This effectively minimises the hydraulic residence time of the organic phase. In an advantageous embodiment of a method according to the present disclosure optimisation of process output (process streams between unit operations) is applied. The unit operations can be two or more biocatalytic conversion units (of which at least one is a reaction zone of the integrated multiphase reactor system) , or combinations of conversion and separation operations. This can be used, e.g., for heat integration, recycle of chemicals, recycle of water, intermediate product conversion (2^{nd}, 3^{rd}, 4^{th} reaction, etc). This can contribute to increased economic feasibility, product recovery, energy efficiency, raw material (e.g. substrate utilisation) and/or minimisation of waste. In an advantageous embodiment, the two or more unit operations comprise operations of two or more biocatalytic reactor units, at least one of which is said biocatalytic reaction zone of the integrated multiphase biocatalytic reactor system and at least one of which is a further biocatalytic reactor unit is a bioreactor vessel having an outlet for a fluid (comprising substrate and optionally biocatalyst and/or produced organic substance) that is connected via a fluid channel with a feed inlet of said biocatalytic reaction zone. An example of an apparatus with such an bioreactor vessel is schematically shown in Figure 13 of WO2021/010822.

Other aspects of the method of the present disclosure, such as a suitable design of the integrated multiphase biocatalytic reactor system comprising a biocatalytic reaction zone, the choice of materials, the production of the organic substance in the reaction zone, the separation in the separation zone, any downstream processing and the like can e.g. be based on WO2021/010822, WO2024049295, of which publications the contents are incorporated herein by reference, or the prior art cited therein.

The method of the present disclosure involves organic substances. The method at least involves the production of a biocatalytically produced substance. Further, the product recovery phase may comprise an additional organic substance, in particular such organic substance for use as an extractant of the organically produced substances. Furthermore, one or more organic substances may be used as an auxiliary liquid, e.g. in down-stream processing. As used herein "organic" refers to any organic substance (such as biocatalytically produced product or an organic liquid phase used for product extraction in the integrated system or for downstream processing) that is chemically oxidisable, as can be determined by the Chemical Oxygen Demand (COD) test, as described in ISO 6060:1989. The biocatalytically produced organic product can be any organic chemical substance or mixture of organic chemical substance that can be produced biocatalytically, in particular fermentatively, in particular any such substance or group of substances mentioned in the prior art cited herein.

The method of the present disclosure is generally particularly advantageous for the recovery of a biocatalytically produced organic substance having a limited solubility in water, due to the substance's hydrophobic and/or lipophilic nature. Typically the aqueous solubility of the produced organic substance is less than 85 g/l, in particular less than 10 g/l, preferably 0-1 g/l, at least at the temperature(s) at which it is contacted with the biocatalytic reaction mixture and/or at ambient temperature (25 degrees C). Advantageously, the biocatalytically produced substance has a higher affinity for a lipid, such as a liquid triglyceride, in particular a vegetable oil (e.g. sunflower oil or castor oil) or a liquid hydrocarbon (e.g. hexane, cyclohexane, pentacosane or the like), relative to water, see also the prior art cited herein.

The organic substance, can be an organic substance having one or more carbon atom and at least one hydrogen atom. Usually, the organic substance, has at least 3 carbon atoms, in particular at least 4 carbon atoms, more in particular at least 5 carbon atoms or at least 6 carbon atoms. The organic substance, in particular the biocatalytically produced product, can have over a 1000 carbons (such as in case of produced biopolymers. In an embodiment, the organic substance has up to 100, up to 50, up to 30 , up to 25 carbons, up to 20 carbons, up to 15 carbons or up to 12 carbons, see also the prior art cited herein.

Advantageously, the method according to the present disclosure can be used to biocatalytically produce one or more organic substances selected from the group consisting of hydrocarbons, in particular monoterpenes, sesquiterpenes, aromatic hydrocarbons, in particular C5-C24 aromatic hydrocarbons, more in particular C6-C12 aromatic hydrocarbons; isoprenoids (terpenoids); organic acids, in particular C5-C24 fatty acids, more in particular C12-C20 fatty acids; alcohols, in particular alcohols having at least 4 carbon atoms, more in particular alcohols having 6-18 or 6-12 carbon atoms ; ketones, in particular ketones having at least 5 carbon atoms, more in particular 6-12 carbon atoms; aldehydes in particular aldehydes having at least 5 carbon atoms, more in particular 6-12 carbon atoms; cyclic carboxylic esters, in particular lactones; non-cyclic esters, in particular non-cyclic esters having at least 5 carbon atoms, more in particular 6-12 carbon atoms; lipids in particular glycerides; ethers; pyridines; imines; imides; amines; amino acids; and peptides. In a preferred embodiment, the method of the present disclosure is used to biocatalytically produce an organic substance selected from the group consisting of 2-phenylethanol, geraniol, linalool, limonene, menthol, carvone, pinene, terpineol, camphine, camphor, indole, farnesene, citronellol, benzaldehyde, butanol, m-cresol, phenol, cinnamaldehyde and squalene.

For further details of preferred organic substances that can in be produced in accordance with the invention, reference is made to the prior art cited herein, in particular WO2021/010822 page 23, line 27 till page 27, line 10 of which the contents are incorporated herein by reference.

Further, a liquid organic substance can be used for the liquid product recovery phase. It is a liquid forming a phase separate from the aqueous reaction phase in the bioreactor system at least in the conditions existing in the reaction zone and the separation zone of the bioreactor system. Typically the aqueous solubility of the extractant is less than 85 g/l, in particular less than 10 g/l, preferably 0-1 g/l, at least at the temperature(s) at which it is contacted with the aqueous reaction phase (such as the fermentation medium) and/or at ambient temperature (25 degrees C). Particularly suitable liquids for the product recovery phase are organic liquids, in particular hydrophobic organic liquids. These may in particular comprise one or more compounds selected from the group of hydrocarbons, organic acids, alcohols, ketones, aldehydes, cyclic carboxylic esters, non-cyclic esters, lipids, amines, including mixtures thereof. Preferred are alkanes, triglycerides and hydrophobic alcohols. Particularly preferred alkanes have 6 carbons (C6) or more, more preferably C7-C25, e.g. C7-C15. Specific examples of particularly suitable liquid alkanes are hexanes, heptanes, octanes, nonanes, decanes, dodecanes and pentacosane. The alkane may be linear or branched or cyclic. Good results have, amongst others been achieved with dodecane. Further, liquid hydrophobic alcohols are particularly suitable. Preferably the alcohol is selected from C8 alcohols and higher, more preferably C10-C20 alcohols. The alcohol may be linear or branched or cyclic. Specific examples of particularly suitable alcohols are an octanol, a decanol, a dodecanol, oleyl alcohol and isomeric mixtures thereof. Further, liquid triglycerides are particularly suitable, preferably vegetable oils. Particularly preferred examples are castor oil, sunflower oil and soy(bean) oil. The various examples of liquids for the recovery phase that are given are not or poorly soluble in water. Furthermore they typically show good biocompatibility, which is of interest when using a living cell for the biocatalysis and using these liquids in the bioreactor system, see also the prior art cited herein.

The term 'biocatalytical(ly)' is generally known in the art to describe methods wherein at least one reaction step in the method is catalysed by a biological material or moiety derived from a biological source, for instance an organism or a biomolecule derived there from. The biocatalyst may in particular comprise one or more enzymes. The biocatalyst may be used in any form. In an embodiment, one or more enzymes are used isolated from the natural environment (isolated from the organism it has been produced in), for instance as a solution, an emulsion, a dispersion, (a suspension of) freeze-dried cells, as a lysate, or immobilised on a support. In an embodiment, one or more enzymes form part of a living organism (such as living whole cells). Particularly good results have been achieved with living cells, in particular with yeast cells and with bacterial cells. Processes wherein the biocatalyst comprises a living cells are also referred to in the art as 'fermentative' methods. The present method can be used for anaerobic processes, aerobic processes and anoxic processes. Advantageously, the biocatalyst comprises a micro-organism selected from the group of bacteria, archaea and fungi, preferably selected from the genera *Pseudomonas, Gluconobacter, Rhodobacter, Clostridium, Escherichia, Paracoccus, Methanococcus, Methanobacterium, Methanocaldococcus* , *Methanosarcina, Aspergillus, Penicillium, Saccharomyces, Kluyveromyces, Pichia, Candida, Hansenula, Bacillus, Corynebacterium, Blakeslea, Phaffia* (*Xanthophyllomyces*), *Yarrowia, Schizosaccharomyces, Zygosaccharomyces, Saccharopolyspora* and *Zymomonas* more preferably from the group of *Corynebacterium glutamicum, Escherichia coli, Bacillus subtilis* , *Bacillus methanolicus, Pseudomonas aeruginosa, Pseudomonas putida, Rhodobacter capsulatus, Rhodobacter sphaeroides, Paracoccus carotinifaciens, Paracoccus zeaxanthinifaciens, Saccharomyces cerevisiae, Saccharomyces pastorianus, Schizosaccharomyces pombe, Aspergillus nidulans, Aspergillus niger, Aspergillus oryzae, Blakeslea trispora, Penicillium chrysogenum, Phaffia rhodozyma* (*Xanthophyllomyces dendrorhous), Pichia pastoris, Yarrowia lipolytica, Saccharopolyspora spinosa and Zymomonas mobilis,* in particular from the group of *Escherichia coli, Pseudomonas aeruginosa, Pseudomonas putida, Saccharomyces cerevisiae, Saccharopolyspora spinosa and Zymomonas mobilis.* A biocatalyst selected from this group may in particular be used for the production of a substance according to the previous paragraph, see also the prior art cited herein.

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise it will be understood that when a connection between structures or components is described, this connection may be established directly or through intermediate structures or components unless specified otherwise. The invention is described more fully hereinafter with reference to the accompanying drawings, in which embodiments of the invention are shown. In the drawings, the absolute and relative sizes of systems, components, layers, and regions may be exaggerated for clarity. Embodiments may be described with reference to schematic and/or cross-section illustrations of possibly idealised embodiments and intermediate structures of the invention. In the description and drawings, like numbers refer to like elements throughout. Relative terms as well as derivatives thereof should be construed to refer to the orientation as then described or as shown in the drawing under discussion. These relative terms are for convenience of description and do not require that the system be constructed or operated in a particular orientation unless stated otherwise.

The term "(at least) substantial(ly)" or "essentially" is generally used herein to indicate that it has the general character or function of that which is specified. When referring to a quantifiable feature, this term is in particular used to indicate that it is at least 50 %, more in particular more than 75 %, even more in particular more than 90 % of the maximum that feature. The term `essentially free' is generally used herein to indicate that a substance is not present (below the detection limit achievable with analytical technology as available on the effective filing date) or present in such a low amount that it does not significantly affect the property of the product that is essentially free of said substance. In practice, in quantitative terms, a product is usually considered essentially free of a substance, if the content of the substance is 0 - 1 wt.%, in particular 0 - 0.5 wt.%, more in particular 0 - 0.1 wt.%.

In the context of this application, the term "about" means generally a deviation of 10 % or less from the given value, in particular a deviation of 5% or less, more in particular a deviation of 2% or less.

For the purpose of clarity and a concise description, features are described herein as part of the same or separate embodiments, however, it will be appreciated that the scope of the invention may include embodiments having combinations of all or some of the features described.

Next the method of the present disclosure will be illustrated by the following example.

### Example: 1 2-phenylethanol (2PE)

A FAST integrated multiphase biocatalytic reactor system (Delft Advanced Biofuels B.V. Delft) was operated in accordance with a method according to the present disclosure for the production of 2-phenylethanol. Model predictions (step a and b) were made for fermentative production of 2PE using *S. Cerevisiae.* A schematic drawing of the used FAST; details about the used *S. Cerevisiae* and starting reaction and separation conditions are found in Brewster et al. 'Inhibition Control by Continuous Extractive Fermentation Enhances De Novo 2-Phenylethanol Production by Yeast' in Biotechnology and Bioengineering, 25 October 2024 (https://doi.org/10.1002/bit.28872). Predicted values of the output variable yield were compared (step c) and ranged from 0.08-0.09 g product/g substrate.

A combination of setpoints of adjustable input variables for manipulating the average residence time was selected (step e) on the basis of the comparison in step c. The dominant selection criteria were a production rate (r_{product}) of about 0.8 g product /kg aqueous phase in the reaction zone per hour for at least 10 days.

This resulted in the following applied setpoints to the system:
1) extractant feed rate was fixed at a ratio for modelled performance at initial feed rate following Fₑₓ = Y_{p/s}, rₛ / (Cₚ, _{Aq} . P).
   Herein
   Fₑₓ = extractant feed rate
   Y_{p/s =} product yield (unit product per unit substrate)
   rₛ = substrate consumption rate
   Cₚ, _{Aq} = product concentration in aqueous phase
   P = partition coefficient
   The design assumption included: Y_{p/s} = 0.08 g/g; P = 18 kg/kg; C_{p,Aq} = 1.0 g/kg.
2) initial feed rate (starting point for RQ control);

The selected combinations of step e were applied to the reactor system, which was operated accordingly. For steps f), g) h):
P-controller required RQ pv, sp was set > pv (RQ_{sp 1.4 1.4} mol/mol with required RQₚᵥ = 1.15 +/- 0.05 mol/mol.

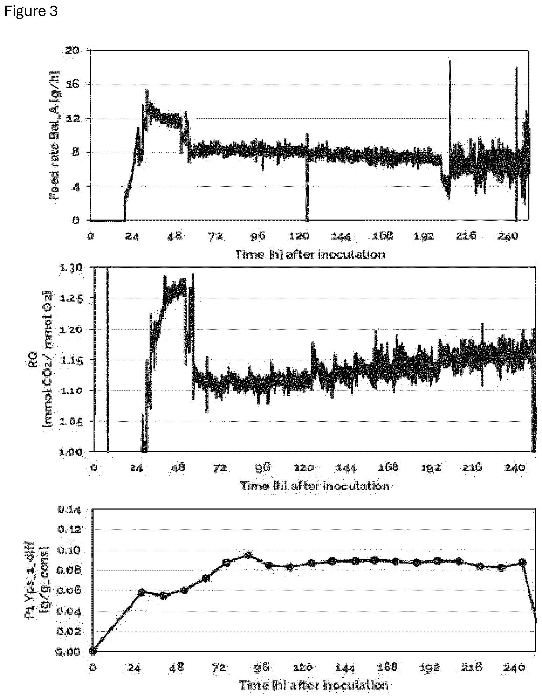

Results are shown in Figure 3.

The skilled person will be able to apply this to methods of the present disclosure for the production of other organic substances, based on the present disclosure, the prior art cited herein and common general knowledge for the biocatalytic production of such other organic substances.

## Claims

1. Method for operating an integrated multiphase biocatalytic reactor system comprising a biocatalytic reaction zone, containing a reaction mixture, wherein an organic substance is biocatalytically produced from a substrate, the system further comprising a separation zone, wherein in said method an output variable of the system, related to the produced organic substance is controlled, the method comprising
- providing the reaction mixture, wherein the organic substance is produced using a biocatalyst, which reaction mixture comprises an aqueous phase, in which the biocatalyst is preferably dispersed or dissolved, which aqueous phase comprises the substrate for the biocatalyst, and wherein further droplets of a liquid product recovery phase, comprising the produced substance, are dispersed in the continuous aqueous phase;
- at least during an essentially steady state stage: continuously our semi-continuously feeding substrate for the biocatalyst into the reaction zone;
- at least during an essentially steady state stage: continuously our semi-continuously transferring reaction mixture from the reaction zone to the separation zone;
- separating the reaction mixture in the separation zone into an upper fluid phase, enriched in the product recovery phase comprising the produced substance and a lower fluid phase, enriched in the aqueous phase and the biocatalyst, compared to the reaction mixture transferred into the separation zone;
- at least during an essentially steady state stage: continuously our semi-continuously selectively withdrawing the enriched product recovery phase from the separation zone or the system;
wherein the control of said output variable comprises
(a) providing a plurality of combinations of setpoints of adjustable input variables for manipulating an average residence time of the produced organic substance in the reaction zone and separation zone,
(b) using a process operating performance model to calculate a predicted value of the output variable for each of said combinations of setpoints of adjustable input parameters,
(c) comparing the predicted values of the output variable,
(d) optionally repeating steps a), b) and c),
(e) selecting a combination of setpoints of adjustable input variables for manipulating the average residence time on the basis of said comparing in step c),
(f) applying the combination of set points of adjustable input variables selected under step e) to the reactor system,
(g) if desired, monitoring the output variable, and
(h) if desired, adjusting one or more of the adjustable input variables setpoint(s) if the value of the monitored output variable deviates from a target value of the output variable or is outside a target value range of the output variable, or repeating one or more of steps a) - g).

2. Method according to claim 1, wherein the provided combinations of setpoints of adjustable process variables in step (a) comprise at least two adjustable process variables selected from the group consisting of: a substrate feed rate, a nutrient or co-feed feed-rate, a water make-up stream, an internal recirculation flowrate, an aeration rate, a rate of discarding reaction mixture rate from the reactor system, a transferring rate of reaction mixture into the separation zone, a withdrawal rate of the enriched product recovery phase from the separation zone, a gas feed rate of the reaction zone (such as with air, oxygen), a characteristic mixing time, a mass transfer resistance between gas to liquid, a mass transfer and mass transfer resistance between liquid-liquid, an addition rate of acid or base, a substrate selection (e.g 1^{st} generation feed, 2^{nd} generation feed, sugars, glucose, glycerol), a concentration and/or composition of surface active chemicals, a water evaporation rate, a liquid level, a gas hold-up, a flow velocity, a height and/or length of a characteristic flow zone, a pressure loss by hydraulic friction, a dispersed phase liquid droplet sizes and distribution, salt (ionic) composition, a salt concentration, a substrate concentration, a selection of an organic liquid for the product recovery phase, a biocatalyst activity, a biocatalyst concentration, a biocatalyst selectivity, a temperature, a pressure, a pH, a conductivity of aqueous broth, an organic liquid phase mass fraction, an organic phase liquid residence time, an aqueous phase residence time, a microbial growth rate, a microbial washout rate ratio of carbon dioxide production rate and oxygen conversion rate, a gas bubble size and gas bubble size distribution, a dissolved substrate or product concentration, a dissolved oxygen concentration, a dissolved carbon dioxide concentration.

3. Method according to claim 1 or 2, wherein at least one controlled output variable is selected from the group consisting of produced organic substance recovery rate (product recovery rate), yield (conversion of substrate to the organic substance), product concentration in the organic phase, microbial (biocatalyst) activity, product recovery, liquid-liquid phase separation/flow velocity in separation zone, oxygen mass transfer rate, microbial productivity, energy efficiency, metabolic flux, raw material utilisation, waste management, turn-around time (minimisation of non-productive time), process safety, process reliability, process stability, microbial biocatalyst substrate conversion rate, microbial biocatalyst growth rate, liquid-liquid phase separation/flow velocities in separation zone, oxygen mass transfer rate, carbon dioxide production rate, oxygen conversion rate, product concentration in the aqueous phase.

4. Method according to claim 3, wherein the controlled output variables at least include the following combinations: microbial substrate conversion rate, biomass growth rate, aqueous phase product concentration and organic phase residence time.

5. Method according to claim 3 or 4, wherein the controlled output variables at least include the following combinations product concentration in the organic phase and microbial activity;

6. Method according to claim 3, 4 or 5, wherein the controlled output variables at least include the following combinations liquid-liquid phase separation rate/flow velocities in the separation zone, oxygen mass transfer rate and microbial activity.

7. Method according to any of the claims 3-6, wherein the controlled output variables at least include the following combinations: biomass concentration, and at least enrichment of one liquid phase in the separation zone and at least one of concentration of surface active chemicals and conductivity;

8. Method according to any of the claims 3-7, wherein the controlled output variables at least include the combination of ratio carbon dioxide production rate and oxygen conversion rate, i.e. respiratory quotient (RQ),and aqueous phase product concentration.

9. Method according to any of the preceding claims, wherein a combination of at least two setpoints of adjustable process variables selected from the group of mass balances related process variables and biocatalytic related process variables are used.

10. Method according to any of the preceding claims, wherein the liquid-liquid phase separation is optimised by controlling the reactor a combination of variables, including one or more of the following: concentration of surface active compounds, concentration of salts, internal flow conditions and power input.

11. Method according to any of the previous claims in which optimisation of process output of process streams between at least two unit operations is applied.

12. Method according to claim 11, wherein the two or more unit operations comprise operations of two or more biocatalytic reactor units, at least one of which is said biocatalytic reaction zone of the integrated multiphase biocatalytic reactor system and at least one of which is a further biocatalytic reactor unit is a bioreactor vessel having an outlet for a fluid (comprising substrate and optionally biocatalyst and/or produced organic substance) that is connected via a fluid channel with a feed inlet of said biocatalytic reaction zone.

13. Method according to claim 11 or 12, wherein the two or more unit operations comprise an operation of said biocatalytic reaction zone of the integrated multiphase biocatalytic reactor system and an operation of said separation zone of the integrated multiphase bioreactor system.

14. Method according to any of the preceding claims, wherein the biocatalytically produced organic substance is selected from the group consisting of 2-phenylethanol, geraniol, linalool, limonene, menthol, carvone, pinene, terpineol, camphine, camphor, indole, farnesene, citronellol, benzaldehyde, butanol, m-cresol, phenol, cinnamaldehyde and squalene.
